# EUROPÄISCHE PATENTSCHRIFT

(11) **EP 0 707 863 B1**
(45) Veröffentlichungstag und Bekanntmachung des Hinweises auf die Patenterteilung: **06.12.2000**
(21) Anmeldenummer: 95114944.2
(22) Anmeldetag: 22.09.1995
(51) Int. Cl.: A61M 16/12, G05D 11/00

(54) **Gasverhältnisregelvorrichtung für Narkosegeräte**
Gas proportions control device for anesthetic apparatus
Dispositif de réglage de la proportion de gaz pour appareil d'anesthésie

(30) Priorität: 18.10.1994 DE 4437207
(43) Veröffentlichungstag der Anmeldung: 24.04.1996
(73) Patentinhaber: DRÄGERWERK AKTIENGESELLSCHAFT, 23542 Lübeck (DE)
(72) Erfinder: Reichert, Dirk-Stefan, D-23554 Lübeck (DE); Götz, Kullik, D-23568 Lübeck (DE); Falb, Wolfgang, D-23628 Krummesse (DE); Schmudde Eckhard, D-23569 Pansdorf (DE); Haase, Thorsten, D-23909 Ratzeburg (DE)

(56) Entgegenhaltungen:
- EP-A- 0 039 932
- DE-A- 3 219 552
- DE-A- 4 111 139
- FR-A- 2 152 521
- GB-A- 2 076 661

## Beschreibung

Die Erfindung betrifft eine Gasverhältnisregelvorrichtung für Narkosegeräte mit einer Narkosegaszuführungsleitung, welche in Reihenschaltung ein Regelventil mit einer Einlaßkammer und einer Auslaßkammer, ein Narkosegas-Einstellventil und einen ersten Meßwiderstand aufweist und mit einer Sauerstoffzuführungsleitung, welche in Reihenschaltung ein Sauerstoff-Einstellventil und einen zweiten Meßwiderstand hat, mit einem Proportionalelement, welches einen den Staudruck vom ersten Meßwiderstand aufnehmenden, durch eine erste Membran begrenzten ersten Druckraum und einen den Staudruck vom zweiten Meßwiderstand aufnehmenden, durch eine zweite Membran begrenzten zweiten Druckraum besitzt, mit einem die Membranen verbindenden, das Regelventil betätigenden Stößel, welcher durch zumindestens zwei durch einen Vorsprung unterteilte Kammern hindurchgeführt ist, deren Kammervolumen von der Auslenkung der Membranen beeinflußt ist.

Eine Gasverhältnisregelvorrichtung der genannten Art ist aus der DE 41 11 139 A1 bekanntgeworden. Bei der bekannten Gasverhältnisregelvorrichtung wird Narkosegas - hier Lachgas - über eine Narkosegaszuführungsleitung in eine Einlaßkammer eines Regelventils und dann von einer Auslaßkammer des Regelventils über ein Narkosegas-Einstellventil und einen ersten Meßwiderstand zu einem Frischgasausgang geleitet. Als weiteres Gas wird Sauerstoff über eine Sauerstoffzuführungsleitung, ein Sauerstoffeinstellventil und einen zweiten Meßwiderstand ebenfalls zu dem Frischgasausgang geführt, wo sich beide Gasströme vereinigen.

Durch die Gasflüsse entstehen an den Meßwiderständen Staudrücke, welche einem Proportionalelement als Differenzdruck zugeführt werden, wobei das Proportionalelement nach der Höhe der Differenz der beiden Staudrücke das Regelventil in der Narkosegas-Zuführungsleitung so steuert, daß der Narkosegasfluß, d.h. der Lachgasfluß, im Verhältnis zum Sauerstofffluß einen bestimmten Wert nicht überschreitet.

Zur Aufnahme des am ersten Meßwiderstand anfallenden Staudruckes besitzt das Proportionalelement einen ersten Druckraum und zur Aufnahme des am zweiten Meßwiderstand entstehenden Staudruckes einen zweiten Druckraum. In jedem Druckraum befindet sich eine Membran, welche proportional zu dem in dem jeweiligen Druckraum herrschenden Druck ausgelenkt wird. Über eine die Membranen verbindende Stange wird ein Kugelsitzventil im Regelventil betätigt und hierdurch der Narkosegasfluß von der Einlaßkammer in die Auslaßkammer verändert.

Bei Narkoseformen mit sehr kleinen Frischgasflüssen, z.B. einen Sauerstofffluß kleiner 1 Liter/Minute, ergeben sich Einstellschwierigkeiten, die dazu führen können, daß der Narkosegasfluß durch das Regelventil ganz oder teilweise abgesperrt wird, wodurch die Sauerstoffkonzentration im Frischgas auf bis zu 100 Vol.-% ansteigen kann. Die Ursache hierfür liegt im wesentlichen darin, daß die vom ersten Druckraum in die Auslaßkammer führende Stange, welche das Kugelsitzventil betätigt, Reibungskräfte an dem Dichtsitz zwischen dem ersten Druckraum und der Auslaßkammer überwinden muß. Die Reibungskräfte werden auch noch dadurch verstärkt, daß in der Auslaßkammer im ungünstigsten Fall der in der Narkosegas-Zufuhrleitung herrschende Vordruck wirksam sein kann. Da der Druck in der Auslaßkammer auch auf den Dichtsitz zwischen der Auslaßkammer und den ersten Druckraum wirkt, erhöht sich hierdurch die Reibungskraft. Zusätzlich neigen derartige membrangesteuerte Ventile zum Schwingen.

Aus der DE 32 19 552 A1 ist ein Druckmindererventil mit einem Schließkolben bekannt, welcher mit einem in einem Regelzylinder befindlichen Regelkolben und einem in einem Dämpfungszylinder aufgenommenen Dämpfungskolben verbunden ist. Der Regelzylinder wird mit einem den Regelkolben betätigenden Steuerdruck beaufschlagt. Der Dämpfungskolben unterteilt den Dämpfungszylinder in einen oberen Zylinderraum und einen unteren Zylinderraum, welche über eine Laminardrossel miteinander verbunden sind. Durch den gedrosselten Gasaustausch zwischen den beiden Zylinderräumen beidseits des Dämpfungszylinders, wird die Empfindlichkeit des Schließkolbens gegenüber plötzlich auftretenden Druckstößen vermindert. Bei dem bekannten Druckmindererventil ist die Wirksamkeit der Dämpfung allerdings von dem den Regelkolben betätigenden Steuerdruck abhängig, da der untere Zylinderraum und der Regelzylinder über eine Nebenleitung miteinander verbunden sind. Bei der Bewegung des Dämpfungskolbens wird somit der Druckausgleich über die Laminardrossel auch von den Druck- und Flowbedingungen in der Nebenleitung beeinflußt.

Um einen Betrieb der bekannten Gasverhältnisregelvorrichtung auch bei kleinen Gasflüssen zu ermöglichen ist versucht worden, das Regelventil mit einem zuschaltbaren Bypass zu überbrücken, DE-C 38 10 745, jedoch ist hiermit nur eine konstante Zudosierung des Narkosegases zu dem Frischgasstrom möglich, dessen Höhe durch die Drosselstelle der Bypassleitung festgelegt ist.

Der vorliegenden Erfindung liegt die Aufgabe zugrunde, eine Gasverhältnisregelvorrichtung derart zu verbessern, daß eine flowproportionale Zudosierung des Narkosegases zum Frischgas auch bei geringen Sauerstoff-Durchflußwerten möglich ist.

Die Lösung der Aufgabe erfolgt dadurch, daß die Auslaßkammer und der erste Druckraum strömungsmäßig einstückig miteinander verbunden sind, daß das Narkosegas-Einstellventil in Strömungsrichtung vor der Einlaßkammer angeordnet ist und daß ein den Gasaustausch zwischen den Kammern drosselnder Strömungswiderstand vorgesehen ist.

Der Vorteil der Erfindung besteht im wesentlichen darin, daß einerseits durch die geänderte Beschaltung des Regelventils mit dem Narkosegas-Einstellventil vor der Einlaßkammer des Regelventils und dem Wegfall eines Dichtsitzes sowohl Öffnungs- und Reibungskräfte minimiert werden und andererseits durch den Strömungswiderstand zwischen den durch die Membranen begrenzten Kammern eine wirksame Dämpfung von dem das Regelventil betätigenden Stößel realisiert ist. Diese Dämpfung arbeitet unabhängig von den die Membranen auslenkenden Steuerdrücken. Der Strömungswiderstand kann als eine Drosselbohrung innerhalb des Vorsprungs ausgeführt sein und der Stößel wird in einer engen Spielpassung durch den Vorsprung hindurchgeführt, so daß der größte Teil des Gasaustausches über die Drosselbohrung erfolgt. Durch diese Maßnahmen ergibt sich ein gutes Ansprechen des Proportionalelementes auf kleine Differenzdruckunterschiede zwischen dem ersten und dem zweiten Druckraum.

Vorteilhafte Ausgestaltungen der Erfindung sind in den Unteransprüchen angegeben.

In vorteilhafter Weise ist zumindestens eine der Kammern über einen weiteren Strömungswiderstand mit der Umgebung verbunden, so daß ein Gasaustausch zwischen der Kammer und der Umgebung möglich ist. Es können auch beide Kammern über Strömungswiderstände mit der Umgebung in Verbindung stehen. Durch Abstimmung der einzelnen Strömungswiderstände untereinander läßt sich ein gutes Ansprechverhalten des Proportionalelementes bei kleinen Differenzdrücken zwischen den Druckräumen einstellen.

In zweckmäßiger Weise besitzt der Vorsprung zwischen den Kammern eine den Stößel aufnehmende Bohrung und der Durchmesser der Bohrung und der Druckmesser des Stößels sind derart bemessen, daß ein gasführender Ringspalt gebildet ist, welcher als Strömungswiderstand zwischen den Kammern dient.

In vorteilhafter Weise besteht der Stößel aus einem ersten Stößelabschnitt und einem zweiten Stößelabschnitt, wobei der erste Stößelabschnitt zwei an den Membranen anliegende Druckstücke aufweist, welche über einen in der Bohrung geführten Stab miteinander verbunden sind. Der zweite Stößelabschnitt ist ein in einer Spielpassung verschiebbarer Stempel, welcher das Regelventil betätigt. Die Membranen sind aus einem biegeschlaffen Elastomer gefertigt und liegen verschiebbar auf den Druckstücken auf. Auf diese Weise werden Verspannungen, die möglicherweise während der Montage zwischen den Membranen und den Stößelabschnitten auftreten können, vermieden, und es wird die Reibung der Stößelabschnitte innerhalb der Bohrung bzw. der Spielpassung gegenüber einer starren Verbindung von Stößel und Membranen weiter reduziert.

Ein Ausführungsbeispiel der Erfindung ist in der Zeichnung dargestellt und im folgenden näher erläutert.

Es zeigen:
- Figur 1: eine erste Gasverhältnisregelvorrichtung nach dem Stand der Technik,
- Figur 2: eine zweite Gasverhältnisregelvorrichtung nach der Erfindung,
- Figur 3: eine dritte Gasverhältnisregelvorrichtung nach der Erfindung.

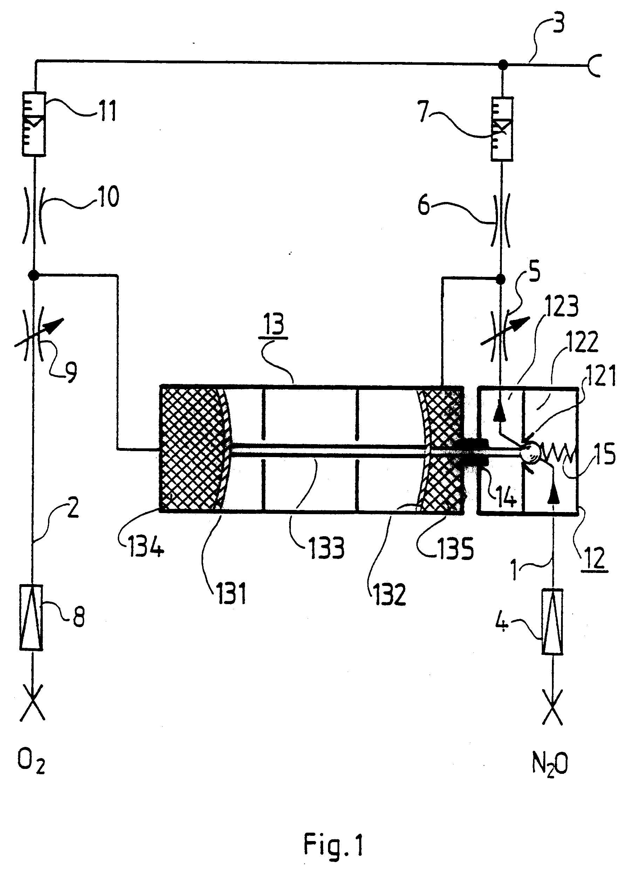

Bei der in der Fig. 1 gezeigten ersten Gasverhältnisregelvorrichtung nach dem Stand der Technik vereinigen sich eine Narkosegas-Zuführungsleitung (1) und eine Sauerstoffzuführungsleitung (2) gemeinsam in einem Frischgasausgang (3). Dem Frischgasausgang (3) nachgeschaltet ist ein Narkosemittelverdunster, welcher in der Fig. 1 nicht dargestellt ist. Die Narkosegas-Zuführungsleitung (1) enthält einen ersten Druckminderer (4), ein Narkosegaseinstellventil (5), einen ersten Meßwiderstand (6) sowie eine erste Durchflußmeßröhre (7). In gleicher Weise sind in der Sauerstoffzuführungsleitung (2) ein zweiter Druckminderer (8), ein Sauerstoffeinstellventil (9), ein zweiter Meßwiderstand (10) und eine zweite Durchflußmeßröhre (11) vorgesehen. In der Narkosegaszuführungsleitung (1) liegt zwischen dem ersten Druckminderer (4) und dem Narkosegaseinstellventil (5) ein Regelventil (12), das über ein erstes Proportionalelement (13) angesteuert wird. Das erste Proportionalelement (13) besteht aus einer ersten Membran (132) und einer zweiten Membran (131), die über einen Stößel (133) miteinander verbunden sind. Die Membranen (132, 131) begrenzen einen ersten Druckraum (135), der mit einem vor dem ersten Meßwiderstand (6) abgegriffenen Narkosegas-Staudruck beaufschlagt ist und einen zweiten Druckraum (134), auf den ein vor dem zweiten Meßwiderstand (10) abgegriffener Sauerstoff- Staudruck wirkt.

Je nach Größe des Sauerstoff-Staudruckes im Verhältnis zum Narkosegas-Staudruck wird der Stößel (133) nach rechts oder links ausgelenkt. Das Regelventil (12) besteht aus einem federbelasteten Kugelsitzventil (121), welches zwischen einer Einlaßkammer (122) und einer Auslaßkammer (123) angeordnet ist, und durch den Stößel (133) zu öffnen oder zu schließen ist. Die Kugel des Kugelsitzventils (121) wird durch eine Feder (15) und durch die zwischen der Einlaßkammer (122) und der Auslaßkammer (123) wirkende Druckdifferenz in den Ventilsitz gedrückt. An der Durchführungsstelle des Stößels (133) zwischen dem ersten Druckraum (135) und der Auslaßkammer (123) befindet sich ein Dichtsitz (14), der sowohl ein Abströmen von Gas in die Umgebung als auch ein gegenseitiges Überströmen zwischen dem ersten Druckraum (135) und der Auslaßkammer (123) verhindert. Zum Öffnen des Kugelsitzventils (121) müssen die Kraft der Feder (15), die aus der Druckdifferenz zwischen Einlaßkammer (122) und Auslaßkammer (123) resultierende Druckkraft auf die Kugel des Kugelsitzventils (121) sowie die durch den Dichtsitz (14) verursachte Reibkraft überwunden werden. Da der Dichtsitz (14) üblicherweise aus einem Elastomer besteht, welches unter dem jeweils herrschenden Druck verformt wird, ist die Reibkraft im allgemeinen druckabhängig. Mit dem Kugelsitzventil (121) kann der Narkosegasstrom in der Narkosegas-Zuführungsleitung (1) gedrosselt oder auch ganz abgesperrt werden. Das Kugelsitzventil (121) befindet sich beispielsweise in der Absperrlage, wenn der Sauerstoff-Staudruck abgefallen ist und die zweite Membran (131) zusammen mit dem Stößel (133) nach links ausgelenkt ist. Durch das Wechselspiel zwischen dem Sauerstoff-Staudruck und dem Narkosegas-Staudruck wird die Zufuhr von Narkosegas geregelt und sichergestellt, daß die Sauerstoff-Mindestkonzentration nicht unter z.B. 25 % Volumenkonzentration absinken kann.

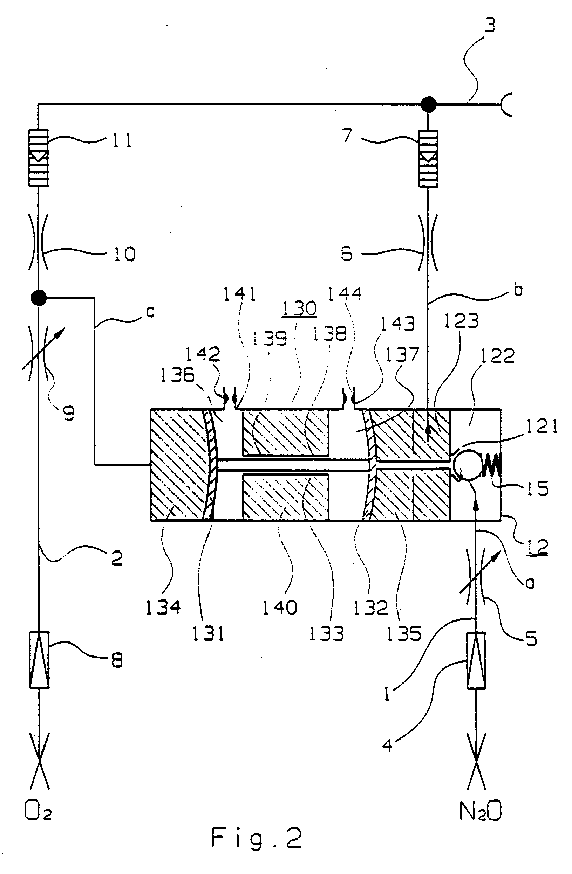

Figur 2 zeigt eine zweite Gasverhältnisregelvorrichtung mit einem zweiten Proportionalelement (130) nach der Erfindung. Gegenüber der Figur 1 ist das Narkosegas-Einstellventil (5) strömungsseitig vor der Einlaßkammer (122) angeordnet, und die Auslaßkammer (123) sowie der erste Druckraum (135) sind einstückig miteinander verbunden, wodurch der Dichtsitz (14), Figur 1, und damit die Reibkraft am Dichtsitz, entfällt. Es herrscht somit in der Auslaßkammer (123) der am ersten Meßwiderstand (6) abfallende Staudruck, der größenordnungsmaßig nur im Millibar-Bereich liegt. Durch die Drosselwirkung des Narkosegas-Einstellventils (5) reduziert sich auch der Druck in der Einlaßkammer (122), so daß auf die Kugel des Kugelsitzventils (121) insgesamt gesehen eine gegenüber der Anordnung nach der Figur 1 deutlich geringere resultierende Druckkraft wirkt. Der zwischen den Membranen (131, 132) liegende Raum ist in eine erste Kammer (136) und eine zweite Kammer (137) unterteilt, wobei die Kammern (136, 137) über einen Ringspalt (138) miteinander verbunden sind. Der Ringspalt (138), ist als ein Strömungswiderstand zwischen den Kammern (136, 137) ausgeführt und wird durch den Durchmesserunterschied zwischen einer Bohrung (139) in einem Absatz (140) innerhalb des zweiten Proportionalelementes (130) und dem Stößel (133) gebildet, welcher durch die Bohrung (139) geführt ist. Der Absatz (140) trennt die erste Kammer (136) von der zweiten Kammer (137). Die erste Kammer (136) ist über einen ersten Auslaß (141) und einen ersten Strömungswiderstand (142) mit der Umgebung verbunden, und die zweite Kammer (137) weist einen entsprechenden zweiten Auslaß (143) mit einem zweiten Strömungswiderstand (144) auf.

Durch den Gasaustausch zwischen den Kammern (136, 137) über den Ringspalt (138) einerseits und den Gasaustausch zwischen den Kammern (136, 137) über die Strömungswiderstände (142, 144) mit der Umgebung andererseits, wird die axiale Bewegung des Stößels (133) gedämpft. Durch Abstimmung der Strömungswiderstände (142, 144) gegenüber dem Ringspalt (138), läßt sich das dynamische Verhalten des zweiten Proportionalventils (130) beim Einstellen auf einen neuen Wert optimieren. Bei dem in der Figur 2 dargestellten zweiten Proportionalelement (130) sind die Membranen (131, 132) direkt mit dem einstückigen Stößel (133) verbunden.

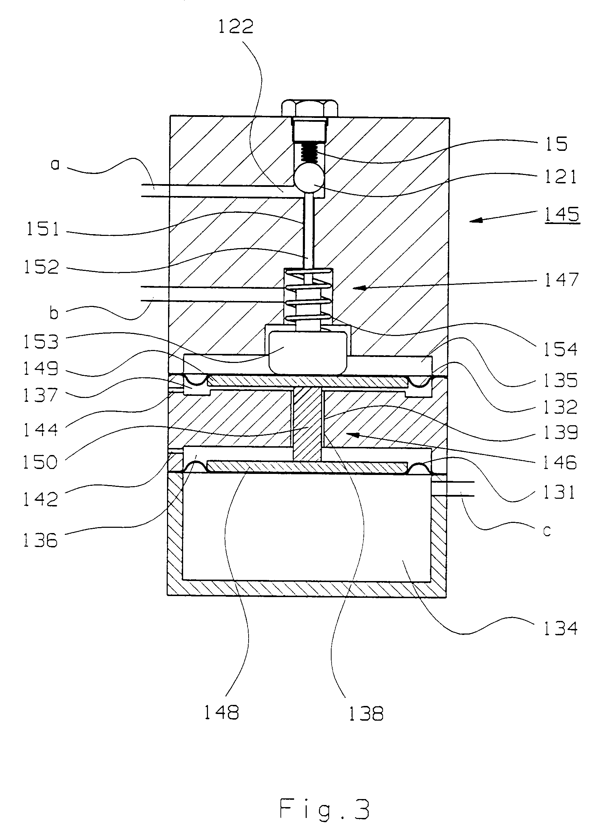

Figur 3 zeigt ein drittes Proportionalelement (145), welches gegenüber dem zweiten Proportionalelement (130) weitere vorteilhafte Verbesserungen besitzt. Gleiche Komponenten sind mit gleichen Bezugsziffern der Figur 2 bezeichnet. Unterschiedlich gegenüber der Figur 2 ist, daß der Stößel (133) in einen ersten Stößelabschnitt (146) und einen zweiten Stößelabschnitt (147) unterteilt ist. Der erste Stößelabschnitt (146) besteht aus zwei scheibenförmigen Druckstücken (148, 149), welche mittels eines durch die Bohrung (139) geführten Stabes (150) miteinander verbunden sind. Der Durchmesser der Bohrung (139) und des Stabes (150) sind derart bemessen, daß der den Strömungswiderstand bildende Ringspalt (138) entsteht. Die Membranen (131, 132) bestehen aus einem biegeschlaffen Elastomer, liegen flächig auf den Druckstücken (148, 149) auf und sind gegenüber diesen verschiebbar. Der zweite Stößelabschnitt (147) ist ein in einer Spielpassung (151) geführter Stempel (152), welcher ein sich gegen die zweite Membran (132) abstützendes Druckstück (153) besitzt. Über die Spielpassung (151) besteht eine Gasverbindung zwischen der Einlaßkammer (122) und dem ersten Druckraum (135). Der Stempel (152) ist gegenüber dem Gehäuse des dritten Proportionalelementes (145) mittels einer Druckfeder (154) abgestützt. Durch die Aufteilung des Stößels in den ersten Stößelabschnitt (146) und den zweiten Stößelabschnitt (147) werden einerseits Reibungseinflüsse, insbesondere des Stabes (150) innerhalb der Bohrung (139) weiter reduziert, da die Membranen (131, 132) gegenüber den Druckstücken (148, 149) verschiebbar sind und dadurch keinerlei mechanische Verspannungen zwischen den Druckstücken (148, 149) und den Membranen (131, 132) auftreten können. Außerdem wird die Montage wesentlich vereinfacht, da der Stößel nicht mehr mechanisch mit den Membranen (131, 132) verbunden werden muß, wie dieses in den Figuren 1 und 2 veranschaulicht ist. Das dritte Proportionalelement (145) hat Anschlüsse (a, b, c), die mit den zugehörigen Leitungen (a, b, c) des Anschlußplanes der Figur 2 verbunden sind.

## Patentansprüche

1. Gasverhältnisregelvorrichtung für Narkosegeräte, mit einer Narkosegaszuführungsleitung (1), welche in Reihenschaltung ein Regelventil (12) mit einer Einlaßkammer (122) und einer Auslaßkammer (123), ein Narkosegas-Einstellventil (5) und einen ersten Meßwiderstand (6) aufweist, und mit einer Sauerstoffzuführungsleitung (2), welche in Reihenschaltung ein Sauerstoff-Einstellventil (9) und einen zweiten Meßwiderstand (10) hat, mit einem Proportionalelement (13), welches einen den Staudruck vom ersten Meßwiderstand (6) aufnehmenden, durch eine erste Membran (132) begrenzten ersten Druckraum (135) und einen den Staudruck vom zweiten Meßwiderstand (10) aufnehmenden, durch eine zweite Membran (131) begrenzten zweiten Druckraum (134) besitzt, mit einem die Membranen (131, 132) verbindenden, das Regelventil (12) betätigenden Stößel (133), welcher durch zumindestens zwei, durch einen Vorsprung (140) unterteilte Kammern (136, 137) hindurchgeführt ist, deren Kammervolumen von der Auslenkung der Membranen (131, 132) beeinflußt ist, dadurch gekennzeichnet, daß die Auslaßkammer (123) und der erste Druckraum (135) strömungsmäßig einstückig miteinander verbunden sind, daß das Narkosegas-Einstellventil (5) in Strömungsrichtung vor der Einlaßkammer (122) angeordnet ist, und daß ein den Gasaustausch zwischen den Kammern (136, 137) drosselnder Strömungswiderstand (138) vorgesehen ist.

2. Gasverhältnisregelvorrichtung nach Anspruch 1, dadurch gekennzeichnet, daß zumindestens eine der Kammern (136, 137) einen über Strömungswiderstände (142, 144) führenden Auslaß (141, 143) zur Umgebung besitzt.

3. Gasverhältnisregelvorrichtung nach Anspruch 1 oder 2, dadurch gekennzeichnet, daß der Vorsprung (140) eine den Stößel (133) aufnehmende zylindrische Bohrung (139) aufweist und daß der Strömungswiderstand durch einen zwischen der Bohrung (139) und dem Stößel (133) befindlichen Ringspalt (138) gebildet ist.

4. Gasverhältnisregelvorrichtung nach Anspruch 3, dadurch gekennzeichnet, daß der Stößel aus einem ersten Stößelabschnitt (146) und einem zweiten Stößelabschnitt (147) besteht, daß der erste Stößelabschnitt (146) aus zwei jeweils an den Membranen (131, 132) anliegenden Druckstücken (148, 149) und einem die Druckstücke verbindenden, in der Bohrung (139) geführten, zylindrischen Stab (150), mit dem Ringspalt (138) zwischen der Bohrung (139) und dem Stab (150) besteht, und daß der zweite Stößelabschnitt (147), welcher das Regelventil (12) betätigt, als ein in einer Spielpassung (151) verschiebbarer, federbelasteter Stempel (152) ausgeführt ist.

5. Gasverhältnisregelvorrichtung nach Anspruch 4, dadurch gekennzeichnet, daß die Membranen (131, 132) aus einem biegeschlaffen Elastomer bestehen.

## Claims

1. Gas-ratio control device for anaesthesia apparatus, having an anaesthetic-gas supply line (1) which has, connected in series, a control valve (12) with an inlet chamber (122) and an outlet chamber (123), an anaesthetic-gas adjusting valve (5) and a first measuring resistance (6), and having an oxygen-supply line (2) which has, connected in series, an oxygen-adjusting valve (9) and a second measuring resistance (10), having a proportional element (13) which has a first pressure chanter (135) that receives the back pressure from the first measuring resistance (6) and is delimited by a first membrane (132) and a second pressure chamber (134) that receives the back pressure from the second measuring resistance (10) and is delimited by a second membrane (131), having a push rod (133) which connects the membranes (131, 132), actuates the control valve (12) and is guided through at least two chambers (136, 137) that are divided by a projection (140) and the chamber volume of which is affected by the deflection of the membranes (131, 132), characterised in that the outlet chamber (123) and the first pressure chamber (135) are connected together in terms of flow so as to be in one piece, in that the anaesthetic-gas adjusting valve (5) is arranged upstream of the inlet chamber (122) in the direction of flow, and in that a flow resistance (138) is provided that restricts the gas exchange between the chambers (136, 137).

2. Gas-ratio control device according to claim 1, characterised in that at least one of the chambers (136, 137) has an outlet (141, 143) that leads by way of a flow resistance (142) and (144) to the environment.

3. Gas-ratio control device according to claim 1 or 2, characterised in that the projection (140) has a cylindrical bore (139) that receives the push rod (133), and in that the flow resistance is formed by an annular gap (138) located between the bore (139) and the push rod (133).

4. Gas-ratio control device according to claim 3, characterised in that the push rod consists of a first push-rod section (146) and a second push-rod section (147), in that the first push-rod section (146) consists of two thrust pieces (148, 149) resting against the respective membranes (131, 132) and a cylindrical bar (150), which connects the thrust pieces and is guided in the bore (139), with the annular gap (138) between the bore (139) and the bar (150), and in that the second push-rod section (147), which actuates the control valve (12), is constructed as a spring-loaded plunger (152) which can be displaced with a clearance fit (151).

5. Gas-ratio control device according to claim 4, characterised in that the membranes (131, 132) consist of an elastomer which is flexurally slack.

## Revendications

1. Dispositif de régulation des proportions de gaz pour appareils d'anesthésie, comportant une conduite d'alimentation en gaz anesthésiant (1) présentant, en série, une soupape de régulation (12) comprenant une chambre d'admission (122) et une chambre d'évacuation (123), une soupape de réglage du gaz anesthésiant (5) et une première résistance de mesure (6), et comportant une conduite d'alimentation en oxygène (2) pourvue, en série, d'une soupape de réglage de l'oxygène (9) et d'une deuxième résistance de mesure (10), comportant un élément proportionnel (13) présentant une première chambre sous pression (135) exposée à la pression dynamique de la première résistance de mesure (6), délimitée par une première membrane (132), et une deuxième chambre sous pression (134), exposée à la pression dynamique de la deuxième résistance de mesure (10), délimitée par une deuxième membrane (131), comportant un poussoir (133) reliant les membranes (131, 132), actionnant la soupape de régulation (12), qui est introduit à travers au moins deux chambres (136, 137), séparées par une saillie (140), le volume des chambres étant influencé par la déformation des membranes (131, 132), caractérisé en ce que la chambre d'évacuation (123) et la première chambre sous pression (135), du point de vue de l'écoulement, sont reliées l'une à l'autre pour ne former qu'un seul composant, en ce que la soupape de réglage du gaz anesthésiant (5) est située en amont de la chambre d'admission (122), suivant la direction d'écoulement, et en ce qu'est prévue une résistance à l'écoulement (138) étranglant l'échange de gaz entre les chambres (136, 137).

2. Dispositif de régulation des proportions de gaz selon la revendication 1, caractérisé en ce qu'au moins l'une des chambres (136, 137) possède un orifice d'échappement (141, 143), donnant sur l'extérieur, par l'intermédiaire de résistances à l'écoulement (142, 144).

3. Dispositif de régulation des proportions de gaz selon la revendication 1 ou 2, caractérisé en ce que la saillie (140) présente un perçage cylindrique (139) recevant le poussoir (133), et en ce que la résistance à l'écoulement est formée par une fente annulaire (138) située entre le perçage (139) et le poussoir (133).

4. Dispositif de régulation des proportions de gaz selon la revendication 3, caractérisé en ce que le poussoir est constitué d'un premier tronçon de poussoir (146) et d'un deuxième tronçon de poussoir (147), en ce que le premier tronçon (146) est constitué de deux éléments de pression (148, 149), s'appliquant chacun sur les membranes (131, 132), et d'une tige (150) cylindrique, reliant les éléments de pression, guidée dans le perçage (139), la fente annulaire (138) se trouvant entre le perçage (139) et la tige (150), et en ce que le deuxième tronçon de poussoir (147), qui actionne la soupape de régulation (12), est conformé en piston (152), placé sous l'action d'un ressort, susceptible d'être déplacé dans un ajustement avec jeu (151).

5. Dispositif de régulation des proportions de gaz selon la revendication 4, caractérisé en ce que les membranes (131, 132) sont constituées en élastomère flexible et mou.
